# EUROPEAN PATENT APPLICATION

(11) **EP 3 517 627 A1**
(43) Date of publication of application: **31.07.2019**
(21) Application number: 19161185.4
(22) Date of filing: 21.05.2013
(51) Int. Cl.: C12Q 1/6806, C12Q 1/70

(54) **METHOD FOR EVALUATION OF VIABILITY OF VIRUSES WITH LYMPHOTROPISM PROPERTIES**

(30) Priority: 18.06.2012 UZ 1200233
(62) Divisional of application: 13807284.8
(71) Applicant: Mkrtchyan, Ovik Leonardovich, Tashkent 100187 (UZ)
(72) Inventor: Gulyamov, Nariman, 100027 Tashkent (UZ)
(74) Representative: Diehl & Partner GbR

(57) **Abstract**

A method of detection of viruses with lymphotropism properties in a biological material with virus concentration below the EIA and PCR sensitivity thresholds, comprises sampling of the biological material, acquisition of a lymphocyte suspension from a healthy human's blood, adding an equal amount of the biological material, mixing and incubation at 37°C for 6-8 hours, washing-out the lymphocytes from the plasma, destruction of lymphocytes, and subjecting the cytoplasm of lymphocyte to the PCR, with detection of viral RNA or DNA in the cytoplasm indicating the presence of viruses, while the absence of viral RNA or DNA indicates the absence of viruses in the biological sample.

## Description

**The invention relates to methods** of detection of viruses with lymphotropism properties in biological substrates with low concentration of viral particles, and of elimination of false-negative results of EIA and PCR test, and may be used in the medical industry and biotechnology.

The detection of virus in biological substrates by its isolation in cell culture is a well-known method. Viruses isolated by cell culture method are identified by haemadsorption, hemagglutination or indirect immunofluorescence methods. Proper sampling and short-time transportation to the laboratory venue on appropriate media are essential for effective isolation of virus isolation in culture. It preserves virus viability and restricts bacteria and fungi reproduction (Laboratory diagnostic of virus infections, Online: http://www.center-hc.ru/). Many viruses, in particular hepatitis B virus (HBV), hepatitis C virus (HCV) and human immunodeficiency virus (HIV), are anthroponotic viruses affecting human cells only thus causing disease of human solely. There are no experimental models of these infections. Also, there are no cultivated cell cultures, particularly in the republic of Uzbekistan, on which one may adequately study cytopathogenic properties and viability of these viruses *in vitro.* Moreover, because of its complexity the method of isolation of viruses on cell cultures is not used for diagnostic purposes.

The immunological method for detection of viruses in biological material is known as enzyme-linked immunosorbent assay (ELISA) is based on using of specific viral proteins extracted from infected cells or produced by genetic engineering by the detection of antibodies to the number of virus antigens (Ilyina, E.N. et. al. Chronic virus liver diseases "Khronicheskiye virusnye zabolevaniya pecheni" Methodological manual for physicians "Metodicheskoye posobiye dlya vrachej" Moscow, 2001, p. 7-11).

In some virus infections, e.g. HCV, EIA detects the antibodies only, thus substantially restricting the evaluation capability of the progress and activity of infection. Moreover, EIA has sensitivity threshold, below which the detection of virus is impossible.

Most close to declared methods of detection of viruses with lymphotropism properties in the biological materials, virus viability assessment and the exclusion of false-negative results of EIA and PCR is the detection of viral RNA or DNA by sampling of biological material and detection of presence of viral RNA or DNA by polymerase chain reaction (PCR) (Ilyina, E.N. et. al. Chronic virus liver diseases "Khronicheskiye virusnye zabolevaniya pecheni" Methodological manual for physicians "Metodicheskoye posobiye dlya vrachej" Moscow, 2001, p. 7-11).

The method relates to direct methods of the detection of the pathogen in the biological material thus permitting to evaluate the activity of viral process. Positive PCR-reaction confirms the presence of the virus in the liver and in the blood with high probability. PCR of the biological samples (plasma or blood proteins, tissue or organs' biopsy materials) not always allow detecting infection caused by viruses with lymphotropism properties, though such viruses may persist in substantially high concentrations in the lymphoid tissue (false-negative results of PCR) and *vice versa* positive PCR may be obtained without persistence of viruses. Furthermore, PCR has the sensitivity threshold, below which virus presence is not detectable by this method.

The goal of presented invention is increasing of reliability of infection detection caused by viruses with lymphotropism properties, elimination of false-negative results testing the blood for the presence of lymphotropic viruses by EIA and PCR, detection of viruses with lymphotropism properties in the biological material with virus concentration below the threshold of IFA or PCR methods sensitivity.

Assigned goal is being resolved by the evaluation of viability of the viruses with lymphotropism properties by sampling of the biological material, detection of presence of viral RNA or DNA by the polymerase chain reaction (PCR); additionally lymphocytes suspension is obtained from the healthy human blood and the equal amount of biological material is added; the mixture is stirred, and incubated at 37°C for 6-8 hours; washing-out of lymphocytes from the plasma and the lymphocytes is being destructed; lymphocytes' cytoplasm is subjected for PCR test. The detection of viral RNA or DNA in the cytoplasm of lymphocytes indicates preserved viability of viruses; the absence of viral RNA or DNA viruses in the cytoplasm of lymphocytes indicated inactivation of viruses. Plasma or blood serum, biopsy samples of tissue or organs, the washouts from the medical instruments may be used as the biological samples. This method allows assessment of the viability of HBV, HCV or HIV viruses.

Assigned objective is being resolved by elimination of EIA and PCR false-negative results by obtaining blood from patients suspected being infected by lymphotropic viruses, and the 6-8 ml of the blood is being sampled into test tubes, containing 2,0 ml normal saline and 2-3 heparin drops; lymphocytes are separated from the blood and incubated at 37°C for 6-8 hours; lymphocytes are washing-out from the plasma and destruction, and the cytoplasm of lymphocytes is subjected to PCR; the detection of viral RNA or DNA in lymphocytes' cytoplasm indicates the presence of viruses; the absence of viral RNA or DNA in lymphocytes' cytoplasm indicates the absence of viruses in the blood. The content of patient's lymphocyte is subjected to PCR-testing.

It is known that many viruses particularly those of hepatitis B virus (HBV), hepatitis C virus (HCV) and human immunodeficiency virus (HIV) can replicate in mononuclear blood cells, particularly, in the lymphocytes and the macrophages. It is known, that under HBV- and HCV-infections simultaneously cause inflammatory processes in the liver with all subsequent hepatitis, as well as secondary immunodeficiency with various degrees of T- lymphopenia and B-lymphopenia, imbalance of regulatory subpopulations ofT-lymphocytes (T-helpers and T-suppressors), reduction of immune regulatory index (IRI) and dysgammaglobulinemia. The degree and grade of immunodeficiency has no relation with the degree of the pathologic process in the liver. Patients with chronic HBV and HCV infections have different intensity of pathological process in the liver tissue after some time, from weak to expressed, but regardless of this, stable and steady aggravation of secondary immunodeficiency.

The dissociation of degree of liver tissue injury and degree of secondary immunodeficiency in various nosological forms of chronic virus hepatitis supported the idea that the hepatitis and secondary immunodeficiency in HBV and HCV infections are associated, mutually aggravating, but not mutually conditional: that is, HBV and HCV along with hepatotropic property possess expressed lymphotropic property - direct property to cause secondary immunodeficiency. Thr differences in clinical appearances of the liver tissue injuries and degree of immunodeficiency in HBV and HCV infections are due to differences in degree of hepatotropic and lymphotropic properties of these viruses. Namely the differences in degrees of hepatotropic and lymphotropic properties of viruses determine the differences of the pathogenesis, clinical appearances and the pattern of antiviral therapy effect in chronic HBV and HCV infections in various stages of these diseases.

The identity of the lymphotropic properties of HBV and HCV, and HIV besides secondary immunodeficiency forming is confirmed also by commonality of their epidemiological features, mechanism of transfer, the progress of associated opportunistic infection (frequent respiratory diseases, intestinal infections), and particularly the development of the lymphogranulomatosis in different tissues of the organism. The development of lymphoid follicles' clusters, which is the lymphogranulomatosis, in various organs and tissues of the organism is supposed to be intrinsic for viral infections of the lymphoid cell system.

Considering lymphotropic properties of HBV, that regardless of serum titer, HBV can permanently persist in essentially high concentrations in the cytoplasm of lymphoid elements. This phenomenon is used in this method for reliable increasing and elimination of false-negative results by EIA and PCR, and detection of lymphotropic viruses in the biological material with concentration of viral particles below the threshold of test-sensitivity of EIA or PCR.

We used the lymphotropic properties of HBV, HCV and HIV in this evaluation method of virus viability - the ability of these viruses to penetrate and persist intracellularly in the healthy human lymphocytes during their *in vitro* incubation.

The evaluation of viability of viruses with lymphotropism properties, in particular HBV, HCV and HIV, required after long term storage of viruses, for the control of the antiviral efficiency of various disinfecting chemical and physical factors against these viruses, as well as for the control of antiviral therapy.

Below is the description of method for evaluation of viability of viruses with lymphotropism properties is performed as described.

### I. Producing of suspension of viruses with lymphotropism properties

For producing of suspension of viruses, the biological material (plasma or blood serum, biopsy samples of tissue or organs, the wash-outs from medical instruments) is obtained. Then the biological material is subjected to quantitative PCR for the verification for the presence of viruses with lymphotropism properties and quantification of titer of viruses. Virus comprising biological material is kept in the frozen state in the refrigerator at below - 25°C temperature.

### II. Producing of lymphocytes suspension from healthy human subject:

a) healthy volunteers are tested for infection with lymphotropic viruses by EIA. Lymphocytes from healthy people with a negative result for study viruses are used in investigations;
b) to receive a sufficient amount of lymphocyte the blood is taken from ulnar vein in an amount of 20-30 ml in the morning from fasting healthy human subject. Then the blood per 7-8 ml is transferred to the centrifuge tubes containing 2 ml normal saline and 3 heparin drops ("Heparin" concentration of 5000 ME/ml; 3 drops contain 750 ME/ml of heparin). The resulting solution is stirred thoroughly;
c) the lymphocytes are separated from the whole heparin containing blood in ficoll-verografin gradient with density d= 1.077 g/ml according to previously described method (Garib, F.Yu., Gurary, N.I., Garib, V.F. "Sposob opredeleniya subpopulyatsij limfotsitov (Determination method of lymphocytes' subpopulations)" // Rasmij Akhborotnoma. Tashkent, 1995, #1, p. 90 - UZ DP 2426). 2 ml of ficoll-verografin gradient is poured into the clean centrifuge tube, then the heparinized blood lays on its surface and the tube is centrifuged at 1500 RPM for 20 minutes. During centrifugation all blood cells, excluding lymphocytes, penetrate through ficoll-verografin gradient. Blood plasma remains above the gradient. In the border of ficoll-verografin gradient and plasma peculiar turbid ring consisting from pure lymphocytes is formed. The ring with lymphocytes is carefully pumped with a pipette and transferred to the clean centrifugal tube;
d) the lymphocytes are washed-out with 10 ml of normal saline 2-3 times with further centrifugation at 1500 RPM for 20 minutes.
e) after last centrifugation the supernatant is removed. The sediment containing lymphocytes is diluted and re-suspended in 600 µl of normal saline. Lymphocytes suspension may be stored no more than 1 day at temperature + 4°C.

### III. Evaluation of viruses with lymphotropism properties to penetrate and persist intracellularly in the human lymphocytes in vitro.

1) Biological material containing viruses with lymphotropism properties is taken from refrigerator and thawed at room temperature.
2) equal amount (300 µl) of virus containing biological material and the suspension of healthy human lymphocytes is transferred with pipette to the clean centrifugal tube; the contents mixed and placed for the incubation (incubation of viruses with lymphocytes *in vitro*) into the thermostat at +37°C for 6-8 hours. The testing tube is mixed with shacking every 1.5-2 hours.
3) The washing-out of lymphocytes. Testing tube is removed from the thermostat. 6-8 ml of normal saline is added, mixed and centrifuged at 1500 RPM for 20 minutes. The lymphocytes are sediment on the bottom of the tube. The supernatant (mixture of plasma with saline) is entirely removed. The lymphocytes are washed out in normal saline and sediment 2-3 times. After last centrifugation, the supernatant is removed the suspension of the lymphocytes (sediment) is diluted with 500 µl of normal saline and transferred to plastic 1.5 lock tube (Eppendorf tube).
4) Thereafter the tube is placed into the freezer of house grade refrigerator overnight. The lymphocytes are destructed under slow freezing condition.
5) The removal of the membrane of destroyed lymphocytes. Next day the tubes from the freezer thawed at room temperature. Then the membranes of destroyed lymphocytes are removed by centrifugation at 3000 RPM for 30 minutes. Membranes are precipitated on the bottom of the tube and lymphocytes' cytoplasm content remains in the supernatant.
6) The supernatant from the tube is transferred and subjected to quantitative PCR of viral RNA or DNA in the cytoplasm of lymphocytes that previously was in infected patient's plasma.

### IV. Assessment of results.

1. Positive PCR for the presence of viral RNA or DNA in the cytoplasm of lymphocytes indicates the remaining virus viability, i.e. ability to penetrate and persist in human lymphocytes *in vitro.*
2. Negative PCR for the presence of viral RNA or DNA in the cytoplasm of lymphocytes indicates the loss (inactivation) of virus viability, i.e. loss of their ability to penetrate and persist inhuman lymphocytes *in vitro.*

Clamming methods are certified by the following examples:

### Example # 1.

The assessment of viability of viruses with lymphotropism properties

The blood is obtained from ulnar vein from the patient after receiving antiviral therapy for hepatitis C. The plasma separated from whole blood and subjected to the quantitative PCR for the verification of HCV presence and quantification virus titer. The PCR is negative. Tested plasma is kept in the freezer at below -25°C temperature.

Simultaneously the 20-30 ml of blood from healthy human volunteers is obtained in the morning from ulnar vein. The part of blood plasma is subjected to PCR for viruses with lymphotropism properties infection. The lymphocytes from the healthy humans with negative results of testing for infection are used for further investigation. Then the 7-8 ml blood aliquots transferred to the centrifuge tubes containing 2 ml of normal saline and 3 heparin drops ("Heparin" concentration is 5000 ME/ml, 3 drops contain 750 ME/ml of heparin). The solution in the tube is mixed thoroughly. The lymphocytes separated from the whole heparinized blood in ficoll-verografin gradient with d= 1.077 g/ml density according to Garib, F.Yu. *et al* method. 2 ml the ficoll-verografin gradient poured into clean centrifuge tube, heparinized blood lays on the surface of gradient and centrifuged at 1500 RPM for 20 minutes. All blood cells excluding lymphocytes penetrate the ficoll-verografin gradient and sediment under it. The blood plasma located above the gradient. On the border between ficoll-verografin gradient and plasma, the peculiar turbid ring with pure lymphocytes suspension is formed. The ring with lymphocytes carefully sucked with a pipette and transferred to the clean centrifuge tube. The lymphocytes are washed out in normal saline and sediment 2-3 times. After last centrifugation, the supernatant is removed. The sediment containing lymphocytes is diluted with 600 µl saline and re-suspended. The lymphocytes suspension may be stored no more than 1 day at + 4°C temperature.

The testing plasma from the freezer is thawed at room temperature. Equal volumes (300 µl) of plasma and the suspension of lymphocytes are transferred to clean centrifuge tube with pipette, mixed and placed for incubation in thermostat at +37°C temperature for 6-8 hours. The tube is mixed by shaking every 1.5-2 hours.

After incubation the tube is removed from the thermostat. 6-8 ml saline is added, mixed and centrifuged at 1500 RPM for 20 minutes. The lymphocytes sediment on the bottom of the tube. The supernatant (mixture of plasma and normal saline) is removed. Then 2-3 times wash-out in normal saline and the lymphocytes sedimentation is performed *ditto.* After last centrifugation, the supernatant is removed, and suspension of lymphocytes (sediment) is diluted by adding 300 µl of normal saline, and transferred to the 1.5 ml lock tube (Eppendorf tube).

Thereafter lymphocyte membranes are destructed by overnight placing into house grade freezer. Next day the tubes are thawed at the room temperature. Then the membranes of destroyed lymphocytes are removed from suspension by centrifugation of tube at 3000 RPM for 30 minutes. The membranes are precipitated on the bottom of the tubes and lymphocyte cytoplasm contents remain in the supernatant. The supernatant is transferred from the tube and subjected to quantitative PCR for the presence of HCV virus in the cytoplasm of lymphocytes. Positive PCR test for HCV indicates the preservation of the HCM viability and the requirement of further antiviral therapy.

### Example #2.

The liver tissue sampled by liver puncture from a patient, who was given antiviral therapy for hepatitis B. Liver biopsy sample is homogenized in 1.5 ml normal saline; transferred to the centrifuge tube, centrifuged at 1500 RPM for 20 minutes; and the supernatant transferred to the tube. One part of supernatant is subjected to quantitative PCR testing for the presence of HCV virus and quantification of virus titer. PCR test for HCV is positive. The biopsy sample is kept in the freezer in the refrigerator at below -25°C temperature.

The lymphocyte suspension from healthy human is made as described in example #1.

The supernatant from liver biopsy sample homogenate is thawed at room temperature. The equal volumes (300 µl) of the supernatant and lymphocyte suspension is added to the tube by automatic pipette; the resulting solution is mixed and placed for incubation into thermostat at +37°C temperature for 6-8 hours. Testing tube is mixed by shaking every 1.5-2 hours.

The tube is removed from thermostat. 6-8 ml of normal saline is added, mixed and centrifuged at 1500 RPM for 20 minutes. The lymphocytes sediment on the bottom of the tube. The supernatant (mixture of plasma with normal saline) is removed entirely. The supernatant (mixture of plasma and normal saline) is removed. Then 2-3 times wash-out in normal saline and the lymphocytes sedimentation is performed *ditto.* After last centrifugation, the supernatant is removed and suspension of lymphocytes (sediment) is diluted by adding 300 µl of normal saline. Thereafter the destruction of lymphocyte membranes is performed by putting testing tube into house grade freezer overnight.

Thereafter lymphocyte membranes are destructed by overnight placing into house grade freezer. Next day the tubes are thawed at the room temperature. Then the membranes of destroyed lymphocytes are removed from suspension by centrifugation of tube at 3000 RPM for 30 minutes. The membranes are precipitated on the bottom of the tubes and lymphocyte cytoplasm contents remain in the supernatant. The supernatant is transferred from the tube and subjected to quantitative PCR for the presence of HBV virus in the cytoplasm of lymphocytes. Negative PCR for HBV indicates the lost virus viability (inactivation).

### Example #3.

The detection of viruses with lymphotropism properties in biological material with the concentration of virus below EIA and PCR sensitivity threshold.

In blood center, the blood plasma from 6-8 ml of blood is being tested for viruses with lymphotropism properties. One part of plasma is subjected to quantitative PCR for testing the presence of HBV, HCV or HIV viruses and the quantification of virus titer. PCR for the presence of viruses is negative. Tested plasma is stored in the freezer at below -25°C.

The lymphocyte suspension from healthy human subject is performed as described in example # 1.

The testing plasma from the freezer is thawed at room temperature. Equal volumes (300 µl) of plasma and the suspension of lymphocytes is transferred to clean centrifuge tube with automatic pipette, mixed and placed for incubation in thermostat at +37°C for 6-8 hours. The tube is mixed by shaking every 1.5-2 hours.

The tube is removed from thermostat. 6-8 ml of normal saline is added, mixed and centrifuged at 1500 RPM for 20 minutes. The lymphocytes sediment on the bottom of the tube. The supernatant (mixture of plasma with normal saline) is removed entirely. The supernatant (mixture of plasma and normal saline) is removed. Then 2-3 times wash-out in normal saline and the lymphocytes sedimentation is performed *ditto.* After last centrifugation, the supernatant is removed and suspension of lymphocytes (sediment) is diluted by adding 300 µl of normal saline.

Thereafter lymphocyte membranes are destructed by overnight placing into house grade freezer. Next day the tubes are thawed at the room temperature. Then the membranes of destroyed lymphocytes are removed from suspension by centrifugation of tube at 3000 RPM for 30 minutes. The membranes are precipitated on the bottom of the tubes and lymphocyte cytoplasm contents remain in the supernatant. The supernatant is transferred from the tube and subjected to quantitative PCR for detection of HBV, HCV and HIV viruses in the content of lymphocytes' cytoplasm. Positive PCR for HCV indicates the presence of HCV virus in the donor plasma its ineligibility for the transfusion.

### Example #4.

The elimination of EIA and PCR false-negative results.

The 6-8 ml of blood obtained in the morning from fasting donor from the ulnar vein. Whole blood transferred to the tube, subjected to the sedimentation, and the serum is obtained; one part of serum is subjected to PCR for the presence of HBV, HCV or HIV viruses and quantification of virus titer. The PCR tests are negative. The rest of the blood serum is stored in the tube. The lymphocyte suspension from healthy human subject is performed as described in example #1.

The lymphocytes separated and destructed by overnight freezing in house grade refrigerator. Next day the tube is thawed at room temperature. Then the membranes of destroyed lymphocytes are removed from suspension by centrifugation at 3000 RPM for 30 minutes. The membranes precipitate on the bottom of the tube, and lymphocyte cytoplasm contents remain in the supernatant. The supernatant is transferred from the tube and subjected to quantitative PCR for detection of HBV, HCV and HIV viruses in the cytoplasm of lymphocytes. Positive PCR for HBV indicated the presence of HBV in the donor blood.

In standard PCR, the detection rate of HBV and HCV in the Republic is 2.7%. According to the epidemiological data new cases of hepatitis B (HBV) and hepatitis C (HCV) transfer occur due to the transfusion of infected blood or its components in 2.2%-5.6%. To reveal the reasons behind and elimination of HBV infection in recipients the lymphotropic properties of the virus were used.

The serums from 309 donor blood samples were tested by PCR for HBV markers detection rate.

PCR revealed HBV in 6 out of 209 serum samples that estimated 1.94% of all number of donors' sample. The same PCR study (study of lymphocytes content from the same donors) revealed HBV in 17 out of 309 samples, estimated 7.44% of all donors' samples. Thus, the standard PCR testing of blood serum was false-negative in 5.50% of samples, thus being the reason of HBV infection in recipients by transfusion of infected blood or it components.

The implementation of PCR testing of lymphocytes for detection of viruses in blood centers will allow significantly reduction of frequency or eliminate the cases of HBV, HCV and HIV after transfusion of blood or it components.

The present disclosure comprises:
1. A method for evaluation of viability of viruses with lymphotropism properties, comprising sampling of the biological material detection of presence of viral RNA or DNA by polymerase chain reaction (PCR), wherein lymphocyte suspension from healthy human subjects is mixed with an equal amount of biological material and incubated at 37°C for 6-8 hours, lymphocytes washed-out from plasma and destructed, with subjecting of lymphocytes' cytoplasm for PCR, detection of viral RNA or DNA in the cytoplasm of lymphocytes that indicate the viability of viruses while the absence of viral RNA or DNA indicates the inactivation of viruses.
2. The method of item 1, wherein at least one of plasma or blood proteins, biopsy tissues or organs, and medical instrument wash-outs is selected and used as the biological materials.
3. The method of item 1, wherein HBV, HCV or HIV viability is assessed.
4. A method of detection of viruses with lymphotropism properties in the biological material with virus concentration below the EIA and PCR sensitivity thresholds consists of sampling of biological material, wherein acquisition of the lymphocyte suspension from the healthy human blood, adding an equal amount of the biological material, mixing and incubation at 37°C for 6-8 hours, washing-out the lymphocytes from the plasma, destruction of lymphocytes, and subjecting the cytoplasm of lymphocyte to the PCR, with detection of viral RNA or DNA in cytoplasm indicating the presence of viruses, while the absence of viral RNA or DNA indicates the absence of viruses in tested biological sample.
5. The method of item 4, wherein plasma or blood proteins are used as biological materials.
6. The method of item 4, wherein HBV, HCV or HIV viability is assessed.
7. A method of elimination of false-negative EIA and PCR results includes sampling of the blood from patients suspected for infection by lymphotropic viruses, wherein 2.0 ml samples of blood taken into a test tube containing 2.0 ml of normal saline and 2-3 drops of heparin, isolation of lymphocytes from 6-8 ml of blood, incubation at 37°C for 6-8 hours, washing of lymphocytes from the plasma, destruction of lymphocyte, and subjecting the cytoplasm of lymphocytes to PCR, detection of viral RNA or DNA that indicates the presence of viruses in the patient's blood while the absence of viral RNA or DNA indicates the absence of viruses in patient's blood.
8. The method of item 7, wherein cytoplasm of lymphocytes separated from patients' bloods is tested by PCR.

## Claims

1. A method of detection of viruses with lymphotropism properties in a biological material with virus concentration below the EIA and PCR sensitivity thresholds, comprising sampling of the biological material, ***characterized by*** acquisition of a lymphocyte suspension from a healthy human's blood, adding an equal amount of the biological material, mixing and incubation at 37°C for 6-8 hours, washing-out the lymphocytes from the plasma, destruction of lymphocytes, and subjecting the cytoplasm of lymphocyte to the PCR, with detection of viral RNA or DNA in the cytoplasm indicating the presence of viruses, while the absence of viral RNA or DNA indicates the absence of viruses in the biological sample.

2. The method of claim 1, wherein plasma or blood proteins are used as biological material.

3. The method of claim 1, wherein the presence of HBV, HCV or HIV is assessed.
